# EUROPEAN PATENT SPECIFICATION

(11) **EP 4 242 305 B1**
(45) Date of publication and mention of the grant of the patent: **21.01.2026**
(21) Application number: 23184512.4
(22) Date of filing: 14.05.2015
(51) Int. Cl.: C12N 11/02, C12P 7/6454, C12N 11/08, C12P 7/64, C12N 9/20, C12P 7/6458

(54) **PROCESS FOR IMMOBILIZATION OF A LIPASE**
VERFAHREN ZUR IMMOBILISIERUNG EINER LIPASE
PROCÉDÉ D'IMMOBILISATION D'UNE LIPASE

(30) Priority: 20.05.2014 EP 14169180
(43) Date of publication of application: 13.09.2023
(62) Divisional of application: 19156967.2
(73) Proprietor: Bunge Loders Croklaan B.V., 1521 AZ Wormerveer (NL)
(72) Inventor: Bhaggan, Krishnadath, NL-1521 AZ Wormerveer (NL); Ma, Jun, NL-1521 AZ Wormerveer (NL); Miorini, Chiara, 33013 Gemona del Friuli Udine (IT); Taran, Viktoria, 2512 AM Den Haag (NL)
(74) Representative: Potter Clarkson

(56) References cited:
- EP-A1- 2 251 428
- EP-A2- 0 882 797
- WO-A1-94/28118
- WO-A1-99/15689
- US-B1- 6 605 452
- CHAUHAN GHANSHYAM S. ET AL: "Designing acrylamide- and methacrylate-based novel supports for lipase immobilization", JOURNAL OF APPLIED POLYMER SCIENCE, vol. 105, no. 5, 1 January 2007 (2007-01-01), US, pages 3006 - 3016, XP093086411, ISSN: 0021-8995, DOI: 10.1002/app.25018
- HANDAYANI NURRAHMI ET AL: "Immobilization of Mucor miehei Lipase onto Macroporous Aminated Polyethersulfone Membrane for Enzymatic Reactions.", MEMBRANES 12 APR 2012, vol. 2, no. 2, 12 April 2012 (2012-04-12), pages 198 - 213, XP002791058, ISSN: 2077-0375
- NEVENA, Z ET AL: "Immobilization of lipase from Candida rugosa on Sepabeads: the effect of lipase oxidation by periodates", BIOPROCESS BIOSYST ENG., vol. 34, 2011, pages 803 - 810, XP019938990

## Description

### Technical Field

The present invention relates to process for immobilizing a lipase on a support having a functional amino group, a process for producing a triglyceride composition using said immobilized lipase and to the use of the lipase in transesterification reactions.

### Background Art

Lipases (E.C. 3.1.1.3), belonging to the group of enzymes, catalyse specifically ester bonds in tri-, di-, and mono-acy;glycerols to glycerol and fatty acids. They further catalyse other reactions such as interesterifications, esterifications, acidolysis, alcoholysis and aminolysis. The high costs of lipases make enzymatic processes economically unattractive. Immobilization of the lipases is a way to increase the industrial susceptibility of lipases and allows recovery of the lipase protein. Lipases can be immobilized on different supports applying various ways of pretreatment of the support or the lipase.

Nevena et al. (NEVENA, Z. Immobilization of lipase from Candida rugosa on Sepabeads: the effect of lipase oxidation by periodates. Bioprocess Biosyst Eng. 2011, no.34, p.803-810.) describes the use of certain Sepabeads^{®} having either amino functional groups or epoxy groups as suitable support for the immobilization of a non-specific lipase from Candida rugosa. Sepabeads^{®} having amino functional groups needed activation with glutaraldehyde or periodate to show improved activity

Palomo et al. (PALOMO, Jose M, et al. Interfacial adsorption of lipases on very hydrophobic support (octadecyl-Sepabeads): immobilization, hyperactivation and stabilization of the open form of lipases. Journal of Molecular Catalysis B: Enzymatic. 2002, vol.19, no.20, p.279-286.) tested the immobilization of various lipases on very hydrophobic support such as octadecyl-Sepabead^{®}.

US 6605452 describes a lipase preparation containing a surfactant-coated lipase complex immobilised onto an insoluble matrix.

Chauhan Ghanshyam et al., Journal of Applied Polymer Science 2007, vol. 105, no. 5, p. 3006-3016) describes polymeric supports for lipase immobilisation prepared by cross-linking two series of hydrogels based on acrylamide and three methacrylates.

Handayani Nurrahmi et al., Membranes 2012, vol. 2, no. 2, p. 198-213) describes the use of polyethersulfone and aminated polyethersulfone in supports for lipase immobilisation.

EP 0882797 describes an enzymatic preparation process for triglycerides of the ABA-type.

WO 94/28118 describes a process for the immobilisation of enzymes on a hydrophobic support material.

EP 2251428 describes a process for preparing a triglyceride composition comprising reacting triolein with stearic acid in the presence of a lipase from *Rhizopus oryzae.*

Enzyme activity is vulnerable to immobilizations regents such as glutaraldehyde or immobilization support. To secure enzyme stability and activity after immobilization of the enzyme, often non-lipase proteins are added such as hen egg album or bovin serum albumin. However, these animal proteins are known to cause allergic reactions.

There remains a need for a simplified immobilization method without additional activation of the support and the right choice of support which will remain lipase activity and stability such as thermos stability to enable the production of commercially relevant triglyceride compositions.

### Summary of invention

The objective of the present invention is to provide an immobilization processes wherein pre-activation of the support could be avoided and if indeed a hydrophobic support such as Sepabeads^{®} having octadecyl groups (EC-OD) are able to perform transesterification reaction to obtain products of commercial importance. Another aim of the present invention was to provide an immobilization process avoiding treatment with non-lipase protein such as animal derived albumin to secure activity and stability of the lipase to produce triglyceride compositions.

According to the present invention, there is provided a process for immobilizing a 1,3 specific lipase on a support containing a functional amino group in the presence of a surface-active material according to claim 1. The term functional amino group refers to an amino group which is engaged in interacting with or binding to the lipase and optionally, the support.

Further disclosed herein is a process for producing a triglyceride by enzymatic transesterification by using a lipase, which is immobilized on a support having a functional amino group.

Also disclosed herein is the use of the immobilized lipase producing a triglyceride fat composition comprising at least 15% by weight OPO.

The support having a functional amino group can be any support having an amino group such as amino-epoxy, or alkyl amino having a carbon chain of C1-C24, preferably C2-C10. The support consists of a methacrylic polymer. Preferably the polymer forms a matrix.

The mechanism of action between the support and the lipase is either by ionic interaction or chemical binding, wherein the ionic interaction is preferred.

The surfactant can be formed from sugars, (both mono-di-and polysaccharides), polyols (e.g. sorbitan and sorbitol) or polyethylene glycols having molecular weight from 350 to 35000, such as PEG s 600, 1500, 4000. Very suitable non-ionic surfactants are polyoxyethylene sorbitan C8-C24 fatty acid esters, in particular those derived from lauric acid, such as Tween 20^{®} or derived from oleic acid such as Tween 80^{®} .

The surfactant concentration in the aqueous solution should be sufficient to ensure effective loading of the support by the enzyme. Very good results were obtained by applying an aqueous solution with a surfactant concentration of at least 0.01 wt%, preferably 0.01-10, most preferably 0.1-5 wt.%.

An ideal amount of lipase in g to support in g is between 1-20 wt.% by weight, preferably 5-15% by weight.

The contact times applied can vary between wide ranges. Suitably, however, contact times between 1 and 72 hours are applied.

The aqueous lipase solution has preferable a concentration between 1 to 20 g/l.

Although the lipase enzyme can be any prior art lipase, a preference is expressed for a lipase which is selected from 1) 1,3-specific lipases from *Rhizomucor miehei* and *Rhizopus oryzae and Thermomyces lanuginosus 2*) lipases from *Penicillium camembertii* specific for the hydrolysis of partial glycerides, preferably Amano G, and 3) lipases specific for the hydrolysis of esters or triglycerides, preferably a lipase from *Candida rugosa .* In particular preferred is a 1,3- specific lipase from *Rhizopus oryzae* such as Lipase D from Amano.

Immobilization of the lipase can be performed in many different ways. Suitably, the contact between support, lipase and/or surfactant is performed as a batch process, as a continuous process in a fixed bed, as a continuous process in a fluidized bed or in a continuously stirred tank, while the contacting is performed with a continuous motion of the lipase solution.

The immobilized lipase according to the invention can be applied in any enzymatic conversion process, such as hydrolysis of triglycerides, diglycerides or esters, but als the esterification or transesterification of fatty acids or diglycerides or triglycerides. These processes are also part of our invention, with the prerequisite that an immobilized lipase according to our invention be used in the process.

Preferred processes for making triglyceride is the production of triglycerides compositions comprising symmetrical triglycerides of the general formula ABA, such as OPO or SOS, wherein O is oleic acid, P is palmitic acid and S is a saturated fatty acid selected from palmitic acid and stearic acid. A particular preferred triglyceride composition of the invention comprises at least 15% by weight OPO.

Triglyceride fats and oils are important commercial products and are used extensively in, for example, the food industry. Some triglycerides are nutritionally important and the triglyceride 1,3-dioleoyl-2-palmitoyl glyceride (OPO) is known to be an important component of human milk fat.

### Examples

The following non-limiting examples illustrate the invention and do not limit its scope in any way. In the examples and throughout this specification, all percentages, parts and ratios are by weight unless indicated otherwise.

### Example 1: Various Sepabeads^{®} with aqueous Lipase D preparation

Preparation of the lipase solutions: Seven lipase solutions were prepared according to Table 1. Sample N°7 was the control sample. All reagents were mixed at 150 rpm at room temperature between 3 to 24 hours and then centrifuged to receive the immobilized lipase as a pellet.

**Table 1**

| Sample, N° | Sepabeads^{®} (functional group) | Amount of Sepabeads in g | Lipase in g |
|---|---|---|---|
| 1 | EC-HA (Hexamethylami no) | 1.5 | 0.12 in 70 ml |
| 2 | EC-OD (Octadecyl) | 1.5 | 0.12 in 70 ml |
| | | | |
| 3 | EC-BU (Butyl) | 1.5 | 0.12 in 70 ml |
| 4 | EC-HFA (Amino-Epoxy) | 1.5 | 0.12 in 70 ml |
| 5 | EC-EA (Ethylamino) | 1.5 | 0.12 in 70 ml |
| 6 | EC-EP (Epoxy) | 1.5 | 0.12 in 70 ml |
| 7 | No support | 0 | 0.18 in 75 ml (equal to 0.12 in 70 ml) |

### Example 2 (comparative)

The acidolysis reaction was performed at 60 °C with all seven lipase preparations using the following acidolysis assay:
1 g Immobilized enzyme (use the pellet after centrifugation)
35 g PO stearin fraction (Feedstock)
49 g Oleic acid
0.126 g H₂O
Composition Feedstock to be found in Table 2.

**Table 2**

| | Feedstock |
|---|---|
| Carbon number | |
| C48 | 62.1 |
| C50 | 24.3 |
| C52 | 8.6 |
| C54 | 1.9 |
| C56 | 0.0 |

The carbon number was determined by GC according to AOCS Ce 5.86.

Table 3 provides the results of the various acidolysis reaction of feedstock after 24 h

**Table 3**

| | HA | OD | BU | HFA | EA | EP | Control |
|---|---|---|---|---|---|---|---|
| Carbon number | | | | | | | |
| C48 | 59.9 | 60.1 | 60.0 | 57.4 | 60.3 | 60.1 | 60.3 |
| C50 | 25.4 | 25.3 | 25.3 | 26.8 | 25.1 | 25.3 | 25.2 |
| C52 | 9.2 | 9.2 | 9.2 | 10.2 | 9.1 | 9.2 | 9.1 |
| C54 | 2.1 | 2.1 | 2.2 | 2.5 | 2.1 | 2.1 | 2.1 |
| C56 | 0.2 | 0.1 | 0.2 | 0.2 | 0.2 | 0.2 | 0.2 |

After 24 h nearly no product OPO or OOP (C52) was produced for all lipase preparations

### Example 3

70 ml of the lipase preparation of Example 1 was mixed with 2.4 g hen egg albumin, 0.65 g Tween 20^{®} and 1.5 g of the respective supports. The acidolysis reaction was preformed according to example 2.

Table 4 shows the results after acidolysis (24 hours) by using various sepabeads with aqueous lipase D solution in the presence of hen egg albumin and TWEEN 20.

**Table 4**

| | HA | OD | BU | HFA | EA | EP |
|---|---|---|---|---|---|---|
| Carbon | | | | | | |
| number | | | | | | |
| C48 | 7.2 | 9.5 | 61.2 | 6.9 | 7.2 | 61.4 |
| C50 | 29.3 | 31.3 | 24.8 | 29.0 | 25.0 | 24.7 |
| C52 | 41.5 | 41.5 | 8.8 | 42.8 | 42.3 | 8.8 |
| C54 | 20.4 | 16.9 | 2.0 | 20.4 | 20.7 | 1.9 |
| C56 | 0.5 | 0.4 | 0.0 | 0.5 | 0.4 | 0.0 |

### Example 4

70 ml of the lipase preparation of Example 1 was mixed with 250 mg PEG 1500, 0.65 g Tween 20^{®} and 1.5 g of the respective supports. The acidolysis reaction was preformed according to example 2. As comparison immobilization on polypropylene (Accurel) under same reaction conditions was performed.

Table 5 shows the results after acidolysis (24 hours) by using various sepabeads with aqueous lipase D solution in the presence of PEG 1500 and Tween 20^{®} .

**Table 5**

| | HA | OD | HFA | EA | Accurel |
|---|---|---|---|---|---|
| Carbon number | | | | | |
| C48 | 6.7 | 61.9 | 6.8 | 6.8 | 58.2 |
| C50 | 27.3 | 24.4 | 27.6 | 27.3 | 25.2 |
| C52 | 42.4 | 8.5 | 42.5 | 42.4 | 10.0 |
| C54 | 22.5 | 1.9 | 22.0 | 22.4 | 2.3 |
| C56 | 0.2 | 0.0 | 0.5 | 0.4 | 4.1 |

### Example 5

Multiple usage of Lipase D immpbilized on support EC-HA.

70 ml of the lipase preparation of Example 1 was mixed with 30 mg PEG 600, 0.65 g Tween 20^{®} and 1.5 g of support EC-HA. The acidolysis reaction was preformed according to example 2. After 3.5 hours the acidolysis reaction was stopped and the immobilized lipase separated from the reaction mixture by filtration. The immobilized lipase is collected and used for the second run of the acidolysis assay . These runs were repeated eight times. At each run a sample (~ 2 ml) at time 3.5 hours were taken for carbon number analysis.

Table 6 shows the results after acidolysis by reusing the immobilized lipase D on EC-HA support in subsequent 8 runs

**Table 6**

| | Run 1 | Run 2 | Run 3 | Run 4 | Run 5 | Run 6 | Run 7 | Run 8 |
|---|---|---|---|---|---|---|---|---|
| C46 | 0.8 | 1.2 | 1.3 | 1.5 | 1.4 | 1.5 | 1.5 | 1.6 |
| C48 | 11.2 | 19.6 | 22.7 | 25.3 | 24.7 | 26 | 27.7 | 28.5 |
| C50 | 34.1 | 35.9 | 36.4 | 35.9 | 35.8 | 35.6 | 35.3 | 35.2 |
| C52 | 42.4 | 34.2 | 31.3 | 29.4 | 30.1 | 29.1 | 28 | 27.4 |
| C54 | 11.2 | 9.1 | 8.2 | 7.8 | 8 | 7.8 | 7.6 | 7.3 |

### Example 6

Lipase D solution (0.9 g/77 ml) was mixed with various Tween in amounts provided in Table 7 and stirred for 15 min. To each of the preparations 1,5 g of Sepabead EC-HA was added and the mixture was stirred for 24 hours. Then immobilized enzyme was filtered off and tested in the acidolysis reaction as described in example 2. Table 7 shows the results of 5 different Tween's after acidolysis after 3.5 h.

**Table 7**

| Carbonnumber | Tween 20 | Tween 40 | Tween 60 | Tween 80 | Tween 85 |
|---|---|---|---|---|---|
| Amount in g | 0.650 | 0.676 | 0.693 | 0.694 | 0.974 |
| C46 | 1.5 | 2.14 | 2.79 | 1.34 | 2.4 |
| C48 | 16.6 | 41.19 | 54.37 | 18.02 | 46.5 |
| C50 | 34.2 | 31.4 | 27.49 | 33.26 | 30.5 |
| C52 | 36.9 | 18.73 | 11.85 | 36.11 | 15.8 |
| C54 | 10.6 | 5.78 | 3.48 | 10.77 | 4.5 |
| C56 | 0.3 | 0.53 | 0 | 0.38 | 0.3 |

### Example 7

Example 6 was repeated with Tween 80^{®} with the difference that the premixing of the lipase solution with Tween 80^{®} was skipped. Lipase solution, Tween 80^{®} and support material were put together and the mixture was stirred for 24 hours. Then immobilized lipase was filtered off and tested in the acidolysis reaction as described in example 2. Table 8 shows the results after acidolysis after 3.5 h.

**Table 8**

| Carbonnumber | With premixing | No premixing |
|---|---|---|
| C46 | 1.3 | 1.51 |
| C48 | 13.8 | 16.38 |
| C50 | 33 | 32.32 |
| C52 | 40.2 | 38.34 |
| C54 | 11.4 | 11.14 |
| C56 | 0.3 | 0.3 |

The results demonstrate that premixing of Tween 80^{®} with lipase solution is not required.

## Claims

1. A process for immobilizing a 1,3 specific lipase on a support containing a functional amino group, wherein the support consists of a methacrylic polymer, which comprises contacting the lipase with said support in the presence of a surface-active material in an aqueous solution, wherein the surface-active material is a non-ionic surfactant, wherein the functional amino group is an ethyl amino group or a hexyl amino group.

2. Process according to claim 1, wherein the 1,3 specific lipase is from *Rhizomucor miehei, Rhizopus oryzae* and *Thermomyces lanuginosus.*

3. Process according to claim 1 or claim 2, wherein the 1,3 specific lipase is from *Rhizopus oryzae.*

4. Process according to any of the preceding claims, wherein the aqueous solution has a concentration of surface-active material of 0.1-5 wt.%.

5. Process according to any of the preceding claims, wherein the amount of lipase in g to support in g is between 5-15% by weight.

6. Process according to any of the preceding claims, wherein the surface-active material is polyoxyethylene sorbitan C8-C24 fatty acid ester.

7. Process according to Claim 6, wherein the surface-active material is derived from lauric acid or derived from oleic acid.

8. Process according to Claims 1 to 5, wherein the surface active material is a mixture of polyethyleneglycol and polysorbate.

9. A process for producing a triglyceride having a symmetrical structure ABA, which process comprises:
immobilizing a lipase according to any of the preceding claims; and
producing the triglyceride by enzymatic transesterification using the immobilized lipase.

10. Process according to Claim 9, wherein the symmetrical triglyceride is OPO, wherein O is oleic acid and P is palmitic acid.

11. Process according to Claim 9, wherein the symmetrical triglyceride is SOS, wherein O is oleic acid and S is a saturated fatty acid selected from palmitic acid and stearic acid.

12. Process according to Claim 9 or 10, wherein the change of C52 triglycerides of feedstock to product is at least 15% by weight.

13. Use of an immobilized lipase obtainable by a process according to any one of claims 1 to 8 for producing a triglyceride fat composition comprising at least 15% by weight OPO, wherein O is oleic acid and P is palmitic acid.

## Patentansprüche

1. Prozess zur Immobilisierung einer 1,3-spezifischen Lipase auf einer Stütze, die eine funktionelle Aminogruppe enthält, wobei die Stütze aus einem Methacrylpolymer besteht, welcher das Inkontaktbringen der Lipase mit der Stütze in Gegenwart eines oberflächenaktiven Materials in einer wässrigen Lösung umfasst, wobei das oberflächenaktive Material ein nichtionisches Tensid ist, wobei die funktionelle Aminogruppe eine Ethylaminogruppe oder eine Hexylaminogruppe ist.

2. Prozess nach Anspruch 1, wobei die 1,3-spezifische Lipase aus *Rhizomucor miehei, Rhizopus oryzae* und *Thermomyces lanuginosus* stammt.

3. Prozess nach Anspruch 1 oder 2, wobei die 1,3-spezifische Lipase aus *Rhizopus oryzae* stammt.

4. Prozess nach einem der vorstehenden Ansprüche, wobei die wässrige Lösung eine Konzentration an oberflächenaktivem Material von 0,1-5 Gew.-% aufweist.

5. Prozess nach einem der vorstehenden Ansprüche, wobei die Menge der Lipase in g zur Stütze in g zwischen 5-15 Gew.-% liegt.

6. Prozess nach einem der vorstehenden Ansprüche, wobei das oberflächenaktive Material Polyoxyethylen-Sorbitan-C8-C24-Fettsäureester ist.

7. Prozess nach Anspruch 6, wobei das oberflächenaktive Material von Laurinsäure abgeleitet ist oder von Ölsäure abgeleitet ist.

8. Prozess nach Anspruch 1 bis 5, wobei das oberflächenaktive Material eine Mischung aus Polyethylenglykol und Polysorbat ist.

9. Prozess zur Herstellung eines Triglycerids, das eine symmetrische Struktur ABA aufweist, wobei der Prozess Folgendes umfasst:
Immobilisieren einer Lipase nach einem der vorstehenden Ansprüche; und
Herstellen des Triglycerids durch enzymatische Umesterung unter Verwendung der immobilisierten Lipase.

10. Prozess nach Anspruch 9, wobei das symmetrische Triglycerid OPO ist, wobei O Ölsäure ist und P Palmitinsäure ist.

11. Prozess nach Anspruch 9, wobei das symmetrische Triglycerid SOS ist, wobei O Ölsäure ist und S eine gesättigte Fettsäure ist, ausgewählt aus Palmitinsäure und Stearinsäure.

12. Prozess nach Anspruch 9 oder 10, wobei die Veränderung der C52 Triglyceride vom Ausgangsmaterial zum Produkt mindestens 15 Gew.-% beträgt.

13. Verwendung einer immobilisierten Lipase, erhältlich durch ein Verfahren nach einem der Ansprüche 1 bis 8 zur Herstellung einer Triglyceridfettzusammensetzung, die mindestens 15 Gew.-% OPO umfasst, wobei O Ölsäure und P Palmitinsäure ist.

## Revendications

1. Procédé d'immobilisation d'une lipase spécifique 1,3 sur un support contenant un groupe amino fonctionnel, dans lequel le support est constitué d'un polymère méthacrylique, qui comprend la mise en contact de la lipase avec ledit support en présence d'un matériau tensioactif dans une solution aqueuse, dans lequel le matériau tensioactif est un tensioactif non ionique, dans lequel le groupe amino fonctionnel est un groupe amino éthylique ou un groupe amino hexyle.

2. Procédé selon la revendication 1, dans lequel la lipase spécifique 1,3 provient de *Rhizomucor miehei, Rhizopus oryzae* et *Thermomyces lanuginosus.*

3. Procédé selon la revendication 1 ou la revendication 2, dans lequel la lipase spécifique 1,3 provient de *Rhizopus oryzae.*

4. Procédé selon l'une quelconque des revendications précédentes, dans lequel la solution aqueuse a une concentration de matériau tensioactif de 0,1 à 5 % en poids.

5. Procédé selon l'une quelconque des revendications précédentes, dans lequel la quantité de lipase en g par rapport au support en g est comprise entre 5 et 15 % en poids.

6. Procédé selon l'une quelconque des revendications précédentes, dans lequel le matériau tensioactif est un ester d'acide gras de polyoxyéthylène sorbitan C8-C24.

7. Procédé selon la revendication 6, dans lequel le matériau tensioactif est dérivé de l'acide laurique ou de l'acide oléique.

8. Procédé selon les revendications 1 à 5, dans lequel le matériau tensioactif est un mélange de polyéthylène glycol et de polysorbate.

9. Procédé de production d'un triglycéride ayant une structure symétrique ABA, lequel procédé comprend :
l'immobilisation d'une lipase selon l'une quelconque des revendications précédentes ; et
la production du triglycéride par transestérification enzymatique à l'aide de la lipase immobilisée.

10. Procédé selon la revendication 9, dans lequel le triglycéride symétrique est OPO, dans lequel O est l'acide oléique et P est l'acide palmitique.

11. Procédé selon la revendication 9, dans lequel le triglycéride symétrique est SOS, dans lequel O est l'acide oléique et S est un acide gras saturé choisi parmi l'acide palmitique et l'acide stéarique.

12. Procédé selon la revendication 9 ou 10, dans lequel le changement des triglycérides C52 de la matière première au produit est d'au moins 15 % en poids.

13. Utilisation d'une lipase immobilisée obtenue par un procédé selon l'une quelconque des revendications 1 à 8 pour produire une composition de graisse de triglycérides comprenant au moins 15 % en poids d'OPO, dans lequel O est l'acide oléique et P est l'acide palmitique.
